(19) Europäisches Patentamt / European Patent Office / Office européen des brevets

(11) **EP 3 766 574 A1**

(12) **EUROPEAN PATENT APPLICATION**
published in accordance with Art. 153(4) EPC

(43) Date of publication:
**20.01.2021 Bulletin 2021/03**

(21) Application number: **19767687.7**

(22) Date of filing: **12.03.2019**

(51) Int Cl.:
*B01J 21/08* (2006.01)      *B01J 37/00* (2006.01)
*B01J 37/03* (2006.01)      *C01B 33/12* (2006.01)
*C07D 301/14* (2006.01)      *C07B 61/00* (2006.01)

(86) International application number:
**PCT/JP2019/010060**

(87) International publication number:
**WO 2019/176951 (19.09.2019 Gazette 2019/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.03.2018 JP 2018048391**

(71) Applicant: **SUMITOMO CHEMICAL COMPANY
LIMITED
Chuo-ku
Tokyo 104-8260 (JP)**

(72) Inventors:
• **OZAWA, Takuya**
  **Ichihara-shi, Chiba 299-0195 (JP)**
• **OUCHI, Yasuhisa**
  **Ichihara-shi, Chiba 299-0195 (JP)**

(74) Representative: **Winter, Brandl, Fürniss, Hübner,
Röss, Kaiser, Polte - Partnerschaft mbB
Patent- und Rechtsanwaltskanzlei
Alois-Steinecker-Straße 22
85354 Freising (DE)**

(54) **PRODUCTION METHOD FOR TITANIUM-CONTAINING SILICON OXIDE MOLDED ARTICLE, AND TITANIUM-CONTAINING SILICON OXIDE MOLDED ARTICLE**

(57)      Provided are (i) a method for producing a titanium-containing silicon oxide molded body having high catalytic activity and high strength and (ii) a titanium-containing silicon oxide molded body having high catalytic activity and high strength. A method for producing a titanium-containing silicon oxide molded body includes: a raw material mixing step of mixing a templating agent, a silicon source, and a solvent; a molding step of obtaining a molded body; and a templating agent removing step of removing the templating agent from the molded body, the molding step being carried out under a condition of 40°C to 100°C, titanium being introduced to a solid or the molded body in and/or after any of the raw material mixing step, the molding step, and the templating agent removing step.

EP 3 766 574 A1

**Description**

Technical Field

The present invention relates to (i) a method for producing a titanium-containing silicon oxide molded body and (ii) a titanium-containing silicon oxide molded body.

Background Art

A titanium-containing silicon oxide has been known as a catalyst for reacting an olefin with a hydroperoxide to obtain an epoxide. For example, Patent Literature 1 discloses an example of how to produce such a catalyst.

Citation List

[Patent Literature]

[Patent Literature 1]
Japanese Patent Application Publication Tokukai No. 2006-159057

Summary of Invention

Technical Problem

In recent years, a titanium-containing silicon oxide having higher strength has been demanded.
An object to be attained by the present invention is to provide (i) a method for producing a titanium-containing silicon oxide molded body having high catalytic activity and high strength and (ii) a titanium-containing silicon oxide molded body having high catalytic activity and high strength.

Solution to Problem

The present invention provides the following [A] and [B].

[A] A method for producing a titanium-containing silicon oxide molded body, including:

a raw material mixing step of mixing a templating agent, a silicon source, and a solvent to obtain a solid containing the templating agent and a silicon oxide;
a molding step of molding the solid, obtained through the raw material mixing step, to obtain a molded body; and
a templating agent removing step of removing the templating agent from the molded body, obtained through the molding step, to obtain the molded body from which the templating agent has been removed,
in the molding step, the solid obtained through the raw material mixing step being molded under a condition that a temperature of the solid is 40°C to 100°C,
in and/or after any of the raw material mixing step, the molding step, and the templating agent removing step, titanium being introduced into the solid or the molded body.

[B] A titanium-containing silicon oxide molded body produced by a method including:

a raw material mixing step of mixing a templating agent, a silicon source, and a solvent to obtain a solid containing the templating agent and a silicon oxide;
a molding step of molding the solid, obtained through the raw material mixing step, to obtain a molded body; and
a templating agent removing step of removing the templating agent from the molded body, obtained through the molding step, to obtain the molded body from which the templating agent has been removed,
in the molding step, the solid obtained through the raw material mixing step being molded under a condition that a temperature of the solid is 40°C to 100°C,
in and/or after any of the raw material mixing step, the molding step, and the templating agent removing step, titanium being introduced into the solid or the molded body.

Advantageous Effects of Invention

According to an aspect of the present invention, provided are (i) a method for producing a titanium-containing silicon oxide molded body having high catalytic activity and high strength and (ii) a titanium-containing silicon oxide molded body having high catalytic activity and high strength.

Description of Embodiments

A method for producing a titanium-containing silicon oxide molded body in accordance with an aspect of the present invention includes a raw material mixing step, a molding step, and a templating agent removing step. The method for producing a titanium-containing silicon oxide molded body in accordance with an aspect of the present invention may further include a silylating step after the templating agent removing step.

According to the method for producing a titanium-containing silicon oxide molded body in accordance with an aspect of the present invention, it is only necessary to introduce titanium into a solid or a molded body in any of the raw material mixing step, the molding step, the templating agent removing step, and the silylating step. Alternatively, it is only necessary to introduce titanium into the solid or the molded body between the raw material mixing step and the molding step, between the molding step and the templating agent removing step, between the templating agent removing step and the silylating step, or after the silylating step. In other words, introduction of titanium into the solid or the molded body may be carried out in, between, and after any of the raw material mixing step, the molding step, the templating agent removing step, and the silylating step.

As used herein, the expression "introduce titanium into a solid or a molded body" means introducing a group represented by -Si-O-Ti into a silicon oxide contained in the solid or the molded body of the solid, by mixing, with a titanium source, the solid containing the silicon oxide or the molded body of the solid. The solid is a solid containing the templating agent and the silicon oxide. The molded body may be a molded body of the solid, a molded body obtained by removing the templating agent from the molded body of the solid, or a titanium-containing silicon oxide molded body.

The introduction of titanium is preferably carried out before the templating agent removing step is started, and is more preferably carried out in the raw material mixing step. If the introduction of titanium is carried out in the raw material mixing step, the raw material mixing step is a step of mixing a silicon source, the titanium source, and the templating agent.

As another aspect of the introduction of titanium, after the templating agent removing step ends, titanium is introduced into the molded body by mixing, with the titanium source, the molded body obtained through the templating agent removing step.

The introduction of titanium may be carried out by, in a liquid phase, mixing, with the titanium source, the solid or the molded body to which titanium is to be introduced or may be alternatively carried out by, in a gas phase, bringing, into contact with the titanium source, the solid or the molded body to which titanium is to be introduced.

Examples of the titanium source encompass titanium alkoxides, chelate-type titanium complexes, halogenated titaniums, and titanium-containing sulfates. Examples of the titanium alkoxides encompass tetramethyl titanate, tetraethyl titanate, tetrapropyl titanate, tetraisopropyl titanate, tetrabutyl titanate, tetraisobutyl titanate, tetra-(2-ethylhexyl) titanate, and tetraoctadecyl titanate. Examples of the chelate-type titanium complexes encompass titanium(IV) oxyacetylacetonate and titanium(IV) diisopropoxybisacetylacetonate. Examples of the halogenated titaniums encompass titanium tetrachloride, titanium tetrabromide, and titanium tetraiodide. Examples of the titanium-containing sulfates encompass titanyl sulfate.

The raw material mixing step in accordance with an aspect of the present invention is a step of mixing the templating agent, the silicon source, and a solvent to obtain the solid containing the templating agent and the silicon oxide.

The "silicon source" used in the raw material mixing step refers to a silicon oxide and a silicon oxide precursor. Examples of the silicon oxide serving as the silicon source encompass amorphous silicas. Examples of the silicon oxide precursor serving as the silicon source encompass alkoxysilanes, alkyltrialkoxysilanes, dialkyldialkoxysilanes, and 1,2-bis(trialkoxysilyl)alkane. Examples of the alkoxysilanes encompass tetramethyl orthosilicate, tetraethyl orthosilicate, and tetrapropyl orthosilicate. Examples of the alkyltrialkoxysilanes encompass trimethoxy(methyl)silane. Examples of the dialkyldialkoxysilanes encompass dimethoxydimethylsilane. The silicon source may be of a single kind, or a combination of two or more kinds.

In the case that the silicon oxide precursor is used as the silicon source in the raw material mixing step, water is used as a part or whole of the solvent in the raw material mixing step. When the silicon oxide precursor is mixed with water, a part or whole of the silicon oxide precursor is transformed into a silicon oxide.

The "templating agent" used in the raw material mixing step refers to a substance which forms a pore structure in the titanium-containing silicon oxide molded body.

The templating agent used in the raw material mixing step is preferably a surfactant or piperidine in which at least one hydrogen atom may be substituted by a hydrocarbon group having 1 to 10 carbon atoms.

Examples of the surfactant encompass cationic surfactants, anionic surfactants, and nonionic surfactants. Examples of the cationic surfactants encompass: quaternary ammonium compounds containing quaternary ammonium ions; and alkyl amine salts. Examples of the quaternary ammonium compounds containing quaternary ammonium ions encompass tetraalkylammonium hydrochloride, tetraalkylammonium acetate, and tetraalkylammonium hydroxide. Examples of the alkyl amine salts encompass monoalkylamine hydrochloride, monoalkylamine acetate, dialkylamine hydrochloride, dialkylamine acetate, trialkylamine hydrochloride, and trialkylamine acetate. Examples of the anionic surfactants encompass alkylbenzene sulfonic acid and salts thereof, $\alpha$-olefin sulfonic acid sodium salts, alkyl sulfuric acid ester salts, alkyl ether sulfuric acid ester salts, methyltaurine acid, alaninate and salts thereof, ether carboxylic acid and salts thereof, sulfosuccinate, sulfonated oil, polyoxyaklylene ether sulfuric acid ester salts, and soap. Examples of the nonionic surfactants encompass polyalkylene oxide, block copolymers of polyalkylene oxide, and alkylamine.

Among these surfactants, the cationic surfactants and the nonionic surfactants are preferable. Among the cationic surfactants, a quaternary ammonium compound containing a quaternary ammonium ion represented by the following Formula (I) is preferable. Among the nonionic surfactants, an amine represented by the following Formula (II) is preferable.

$$[NR^1R^2R^3R^4]^+ \qquad (I)$$

where: $R^1$ represents a hydrocarbon group having 2 to 36 carbon atoms; and $R^2$ to $R^4$ each independently represent a hydrocarbon group having 1 to 6 carbon atoms.

$$NR^5R^6R^7 \qquad (II)$$

where: $R^5$ represents a hydrocarbon group having 2 to 36 carbon atoms; and $R^6$ and $R^7$ each independently represent a hydrogen atom or a hydrocarbon group having 1 to 6 carbon atoms.

In Formula (I), $R^1$ is a hydrocarbon group having 2 to 36 carbon atoms, preferably a hydrocarbon group having 6 to 36 carbon atoms, and more preferably a hydrocarbon group having 10 to 22 carbon atoms. $R^2$ to $R^4$ are each independently a hydrocarbon group having 1 to 6 carbon atoms, and all of $R^2$ to $R^4$ are each preferably a methyl group.

Specific examples of the quaternary ammonium ion represented by Formula (I) encompass cations of decyl trimethylammonium, dodecyl trimethylammonium, hexadecyl trimethylammonium, octadecyl trimethylammonium, eicosyl trimethylammonium, behenyl trimethylammonium, benzyl trimethylammonium, and dimethyldidodecyl ammonium.

Specific examples of the quaternary ammonium compound containing the quaternary ammonium ion represented by Formula (I) encompass decyl trimethylammonium hydroxide, decyl trimethylammonium chloride, decyl trimethylammonium bromide, dodecyl trimethylammonium hydroxide, dodecyl trimethylammonium chloride, dodecyl trimethylammonium bromide, hexadecyl trimethylammonium hydroxide, hexadecyl trimethylammonium chloride, hexadecyl trimethylammonium bromide, octadecyl trimethylammonium hydroxide, octadecyl trimethylammonium chloride, octadecyl trimethylammonium bromide, eicosyl trimethylammonium hydroxide, eicosyl trimethylammonium chloride, eicosyl trimethylammonium bromide, behenyl trimethylammonium hydroxide, behenyl trimethylammonium chloride, behenyl trimethylammonium bromide, and dimethyl dialkyl ammonium salts and methyl trialkyl ammonium salts each obtained by substituting at least one methyl group in these salts each containing the quaternary ammonium ion for an alkyl group having 2 to 22 carbon atoms.

In Formula (II), $R^5$ is a hydrocarbon group having 2 to 36 carbon atoms, preferably a hydrocarbon group having 6 to 36 carbon atoms, and more preferably a hydrocarbon group having 10 to 22 carbon atoms. $R^6$ and $R^7$ are each independently a hydrogen atom or a hydrocarbon group having 1 to 6 carbon atoms, and $R^6$ and $R^7$ are each preferably a hydrogen atom.

Specific examples of the amine represented by Formula (II) encompass octylamine, nonylamine, decylamine, undecylamine, dodecylamine, tridecylamine, tetradecylamine, pentadecylamine, hexadecylamine, heptadecylamine, octadecylamine, nonadecylamine, eicosylamine, behenylamine, and methylalkylamine and dimethylalkylamine each obtained by substituting at least one hydrogen atom in any of these amines for a methyl group.

Examples of the piperidine in which at least one hydrogen atom may be substituted by a hydrocarbon group having 1 to 10 carbon atoms encompass piperidine, 1-methylpiperidine, 2-methylpiperidine, 2,6-dimethylpiperidine, and 2,2,6,6-tetramethylpiperidine.

The templating agent may be of a single kind, or a combination of two or more kinds.

The templating agent and the silicon source are mixed in the presence of the solvent. Examples of the solvent encompass water and alcohols. Examples of the alcohols encompass methanol, ethanol, 1-propanol, and 2-propanol.

Through the raw material mixing step, the solid containing the templating agent and the silicon oxide is obtained. The solid obtained through the raw material mixing step and containing the templating agent and the silicon oxide is separated, by filtration or the like, from the solvent having been used in the raw material mixing step. The raw material mixing step is preferably carried out for 2 hours to 1000 hours at a temperature ranging from 20°C to 200°C. During mixture, stirring can be also carried out.

Note that a binder and/or the like each of which is generally used may be added, provided that desired performance is not impaired.

The molding step in accordance with an aspect of the present invention is a step of molding the solid, containing the templating agent and the silicon oxide, to obtain the molded body. The molding step is a step from introduction of the solid, containing the templating agent and the silicon oxide, into a device for pressing and molding the solid, to obtainment of the molded body thus pressed. In the molding step, such a molding device for molding the solid is not limited to a molding device including a heating means for adjusting a temperature of the solid, provided that the solid is heated while the solid is pressed and molded. As an example, the molding step may be carried out by (i) heating the solid to a temperature in a range of 40°C to 100°C with use of a preheater and (ii) while maintaining the temperature in the range, introducing the solid into the molding device which does not include a heating means.

As a molding method, any method may be employed such as a compression method, typified by roll press molding (briquetting, compacting), hydraulic press molding, and tablet molding, and extrusion molding. In the extrusion molding, an organic binder and an inorganic binder each of which is generally used can be used. The molding method in the molding step is preferably the compression method, from the viewpoint of catalytic strength and catalytic properties.

In the case that compression molding is carried out, the solid containing the templating agent preferably contains an appropriate amount of water. By compression-molding the solid containing the templating agent in a state where the solid contains an appropriate amount of water, it is possible to produce the molded body which has sufficient strength. A water content of the solid to be compression-molded is preferably 1% by weight to 70% by weight, and more preferably 3% by weight to 40% by weight. An amount of the water may be adjusted by adjusting a dry condition at a time when the solid which is wet is dried or may be alternatively adjusted by adding water to the solid which has been sufficiently dried.

If the roll press molding is employed as an aspect, a pressure applied during the compression molding is typically 0.1 ton/cm to 10 ton/cm, preferably 0.5 ton/cm to 8 ton/cm, and more preferably 1 ton/cm to 6 ton/cm. If the hydraulic press molding or the tablet molding is employed as an aspect, a pressure applied during the compression molding is typically 0.1 ton/cm$^2$ to 10 ton/cm$^2$, preferably 0.2 ton/cm$^2$ to 5 ton/cm$^2$, and more preferably 0.5 ton/cm$^2$ to 2 ton/cm$^2$.

The molded body which is obtained by the press molding or the tablet molding may have any shape such as a shape of a tablet, a sphere, or a ring. The molded body may be used as it is for a reaction and the like or may be alternatively used after the molded body is crushed into pieces each having an appropriate size.

In the molding step, the solid containing the templating agent and the silicon oxide is molded under a condition that the temperature of the solid at a time of molding is preferably 40°C to 100°C, and more preferably 40°C to 70°C. The temperature of the solid at the time of the molding is the temperature of the solid immediately before, immediately after, or during the molding. The temperature of the solid is checked by directly measuring, with use of a contact thermometer, a radiation thermometer, or the like, the temperature of the solid immediately before, immediately after, or during the molding. The temperature at a time of heating in the molding step tends to allow (i) obtainment of a titanium-containing silicon compound molded body which has higher catalytic activity, as the temperature becomes lower in the range of 40°C to 100°C and (ii) obtainment of the titanium-containing silicon compound molded body which has a lower powdering ratio, as the temperature becomes higher in the range of 40°C to 100°C.

In the case that the temperature of the solid at the time of the molding falls within the above range, it is possible to produce the titanium-containing silicon oxide molded body which has high catalytic activity and which has high strength (later described), by (i) molding the molded body containing the templating agent and having a powdering ratio of not more than 3% and (ii) removing the templating agent in a subsequent step. Note that how to evaluate the powdering ratio of the molded body containing the templating agent will be described in detail in the section of "Examples" in the specification.

The temperature of the solid at the time of the molding can be controlled by, for example, a method of, before the molding, preheating the solid to be molded, a method of molding the solid with a heated molding part, or both of these methods. Specifically, in the case that the roll press molding is employed, a method of heating the solid in a raw material feed hopper, a method of heating a roller itself to a given temperature, or the like is employed. In the case that the hydraulic press molding or the tablet molding is employed, a method of heating a mold to a given temperature, a method of preheating the solid to be introduced into the mold, or the like is employed.

The templating agent removing step in accordance with an aspect of the present invention is a step of removing the templating agent from the molded body, obtained through the molding step, to obtain the molded body from which the templating agent has been removed. By carrying out the templating agent removing step, the molded body which does not contain the templating agent or which substantially does not contain the templating agent is obtained.

Removal of the templating agent is achieved by calcinating the molded body, containing the templating agent, at a temperature of 300°C to 800°C in air or by extracting the templating agent with use of a solvent. Note that the templating agent is preferably removed by extraction with use of the solvent.

A technique of extracting a templating agent with use of a solvent is reported by Whitehurst et. al. (see U.S.

Patent No. 5143879). The solvent used for the extraction only needs to be one that is capable of dissolving therein a compound used as the templating agent. Generally, the solvent can be a compound having 1 to 12 carbon atoms and being in a liquid state at an ordinary temperature or can be a mixture of two or more such compounds. Suitable examples of the solvent encompass alcohols, ketones, and acyclic and cyclic ethers and esters. Examples of the alcohols encompass methanol, ethanol, ethylene glycol, propylene glycol, 1-propanol, 2-propanol, 1-butanol, and octanol. Examples of the ketones encompass acetone, diethyl ketone, methyl ethyl ketone, and methyl isobutyl ketone. Examples of the ethers encompass diisobutyl ether and tetrahydrofuran. Examples of the esters encompass methyl acetate, ethyl acetate, butyl acetate, and butyl propionate.

The solvent used for the removal of the templating agent is preferably one that can dissolve therein the templating agent used, from the viewpoint of ability of dissolving the templating agent. For example, in the case that the templating agent is the salt containing the quaternary ammonium ion, the alcohols are preferable, and, in particular, methanol is more preferable. A mass ratio of the solvent for the extraction to the molded body is typically 1 to 1000, and preferably 5 to 300. In order that an effect of extracting the templating agent is enhanced, an acid or a salt thereof may be added to the solvent. Examples of the acid used encompass inorganic acids, such as hydrochloric acid, sulfuric acid, nitric acid, and bromic acid, and organic acids, such as formic acid, acetic acid, and propionic acid. Examples of the salt encompass alkali metal salts, alkaline earth metal salts, and ammonium salts. A concentration of the acid or the salt thereof added in the solvent is preferably not more than 30% by mass, and more preferably not more than 15% by mass.

As a method for removing the templating agent, after the solvent and the molded body are sufficiently mixed, a liquid phase portion is separated by a method such as filtration or decantation. This operation may be repeated multiple times. Alternatively, the templating agent can be extracted in such a way that (i) a container such as a column is filled with the molded body and (ii) the solvent for the extraction is caused to flow through the container. A temperature at which the extraction is carried out is preferably 0°C to 200°C, and more preferably 20°C to 100°C. In the case that a boiling point of the solvent for the extraction is low, the extraction may be carried out under increased pressure.

The templating agent in a solution obtained by an extraction process can be collected and reused as the templating agent in the raw material mixing step. Similarly, the solvent for the extraction can be also reused after the solvent is purified by a typical distillation operation or the like.

The silylating step in accordance with an aspect of the present invention is a step of bringing the molded body, obtained through the templating agent removing step, into contact with a silylating agent to obtain the titanium-containing silicon oxide molded body. By carrying out the silylating step, the molded body obtained through the templating agent removing step is silylated.

Silylation may be carried out by a gas phase method in which the molded body, obtained through the templating agent removing step, and the silylating agent in a gaseous state are brought into contact with each other and thereby caused to react with each other or may be alternatively carried out by a liquid phase method in which, in a solvent, the molded body and the silylating agent are brought into contact with each other and thereby caused to react with each other. In an aspect of the present invention, the liquid phase method is more preferable. Typically, in the case that the silylation is carried out by the liquid phase method, a hydrocarbon is suitably used as the solvent in the silylating step. In the case that the silylation is carried out by the liquid phase method, drying may be carried out thereafter.

As used herein, the silylating agent is a silicon compound which has reactivity with respect to the molded body and in which a hydrolyzable group is bonded to silicon and, to the silicon, at least one group selected from the group consisting of alkyl groups, allyl groups such as vinyl groups, aryl groups such as phenyl groups, halogenated alkyl groups, and siloxy groups is bonded. Examples of the hydrolyzable group bonded to the silicon encompass hydrogen, halogens, alkoxy groups, acetoxy groups, and amino groups. In the silylating agent, a single hydrolyzable group is preferably bonded to the silicon.

Examples of the silylating agent encompass organic silanes, organic silylamines, organic silylamides and derivatives thereof, and organic silazanes.

Examples of the organic silanes encompass chlorotrimethylsilane, dichlorodimethylsilane, chlorobromodimethylsilane, nitrotrimethylsilane, chlorotriethylsilane, iododimethylbutylsilane, chlorodimethylphenylsilane, chlorodimethylsilane, dimethyl n-propylchlorosilane, dimethylisopropylchlorosilane, tert-butyldimethylchlorosilane, tripropylchlorosilane, dimethyloctylchlorosilane, tributylchlorosilane, trihexylchlorosilane, dimethylethylchlorosilane, dimethyloctadecylchlorosilane, n-butyldimethylchlorosilane, bromomethyldimethylchlorosilane, chloromethyldimethylchlorosilane, 3-chloropropyldimethylchlorosilane, dimethoxymethylchlorosilane, methylphenylchlorosilane, methylphenylvinylchlorosilane, benzyldimethylchlorosilane, diphenylchlorosilane, diphenylmethylchlorosilane, diphenylvinylchlorosilane, tribenzylchlorosilane, methoxytrimethylsilane, dimethoxydimethylsilane, trimethoxymethylsilane, ethoxytrimethylsilane, diethoxydimethylsilane, triethoxymethylsilane, trimethoxyethylsilane, dimethoxydiethylsilane, methoxytriethylsilane, ethoxytriethylsilane, diethoxydiethylsilane, triethoxyethylsilane, methoxytriphenylsilane, dimethoxydiphenylsilane, trimethoxyphenylsilane, ethoxytriphenylsilane, diethoxydiphenylsilane, triethoxyphenylsilane, dichlorotetramethyldisiloxane, 3-cyanopropyldimethylchlorosilane, 1,3-dichloro-1,1,3,3-tetramethyldisiloxane, and 1,3-dimethoxy-1,1,3,3-tetramethyldisiloxane.

Examples of the organic silylamines encompass N-trimethylsilylimidazole, N-tert-butyldimethylsilylimidazole, N-dimethylethylsilylimidazole, N-dimethyl-n-propylsilylimidazole, N-dimethylisopropylsilylimidazole, N-trimethylsilyldimethylamine, N-trimethylsilyldiethylamine, N-trimethylsilylpyrrole, N-trimethylsilylpyrrolidine, N-trimethylsilylpiperidine, 1-cyanoethyl(diethylamino)dimethylsilane, and penta fluorophenyldimethylsilylamine.

Examples of the organic silylamides and the derivatives thereof encompass N,O-bis(trimethylsilyl)acetamide, N ,O-bis(trimethylsilyl)trifluoroacetamide, N-(trimethylsilyl) acetamide, N-methyl-N-(trimethylsilyl) acet amide, N-methyl-N-(trimethylsilyl)trifluoroacetamide, N-methyl-N-(trimethylsilyl)heptafluorobutylamide, N-(tert-butyldimethylsilyl)-N-trifluoroacetamide, and N,O-bis(diethylhydrosilyl)trifluoroacetamide.

Examples of the organic silazanes encompass 1,1,1,3,3,3-hexamethyldisilazane, heptamethyldisilazane, 1,1,3,3-tetramethyldisilazane, 1,3-bis(chloromethyl)-1,1,3,3-tetramethyldisilazane, 1,3-divinyl-1,1,3,3-tetramethyldisilazane, and 1,3-diphenyl-1,1,3,3-tetramethyldisilazane.

Further examples of the silylating agent encompass N-methoxy-N,O-bis(trimethylsilyl)trifluoroacetamide, N-methoxy-N,O-bis(trimethylsilyl)carbamate, N,O-bis(trimethylsilyl)sulfamate, trimethylsilyl trifluoromethane sulfonate, and N,N'-bis(trimethylsilyl)urea. The silylating agent is preferably an organic silazane, and more preferably 1,1,1,3,3,3-hexamethyldisilazane.

The titanium-containing silicon oxide molded body produced by an aspect of the present invention can be used as a catalyst for an oxidation reaction of an organic compound, e.g., an epoxidation reaction of an olefin. In particular, the titanium-containing silicon oxide molded body is preferably used in production of an epoxide in which production an olefin and a hydroperoxide are caused to react with each other. That is, a method for producing an epoxide in accordance with an aspect of the present invention preferably includes causing an olefin and a hydroperoxide to react with each other in the presence of a catalyst containing the titanium-containing silicon oxide molded body produced by the above-described method.

The olefin subjected to the epoxidation reaction may be an acyclic olefin, a monocyclic olefin, a bicyclic olefin, or a polycyclic olefin having three or more rings, and may be a monoolefin, a diolefin, or a polyolefin. In the case that the olefin has two or more double bonds in its molecule, the double bonds may be conjugated bonds or may be alternatively non-conjugated bonds. Generally, the olefin preferably has 2 to 60 carbon atoms. The olefin may have a substituent. Examples of such an olefin encompass ethylene, propylene, 1-butene, isobutylene, 1-hexene, 2-hexene, 3-hexene, 1-octene, 1-decene, styrene, and cyclohexene. Further, the olefin may have a substituent(s) containing an oxygen atom, a sulfur atom, or a nitrogen atom together with a hydrogen atom and/or a carbon atom. Examples of such an olefin encompass allyl alcohol, crotyl alcohol, and allyl chloride. Examples of the diolefin encompass butadiene and isoprene. Particularly preferable examples of the olefin encompass propylene.

Examples of the hydroperoxide include organic hydroperoxides. The organic hydroperoxides are each a compound having a structure represented by the following Formula (III):

$$R-O-O-H \qquad (III)$$

where R represents a hydrocarbon group. The organic hydroperoxides each react with an olefin to produce an epoxide and a hydroxyl compound. R in Formula (III) is preferably a hydrocarbon group having 3 to 20 carbon atoms, and more preferably a hydrocarbon group having 3 to 10 carbon atoms. Specific examples of the organic hydroperoxides encompass tert-butylhydroperoxide, 1-phenylethylhydroperoxide, and cumenehydroperoxide. Hereinafter, the cumenehydroperoxide may be abbreviated to CMHP.

In the case that CMHP is used as an organic hydroperoxide, an obtained hydroxyl compound is 2-phenyl-2-propanol. This 2-phenyl-2-propanol produces cumene when subjected to a dehydration reaction and a hydrogenation reaction. Hereinafter, cumene may be abbreviated to CUM. By oxidizing this CUM, CHMP is obtained again. From this viewpoint, CMHP is preferably used as the organic hydroperoxide used for the epoxidation reaction.

The epoxidation reaction can be carried out in a liquid phase with use of a solvent, a diluent, or a mixture thereof. The solvent and the diluent should be each a liquid at a temperature during the reaction and under a pressure during the reaction, and should be substantially inert against a reactant and a product. In the case that CMHP is subjected to the epoxidation reaction in the presence of CUM which is a raw material of CMHP, it is possible to use the CUM as a solvent without adding any particular solvent.

The temperature during the epoxidation reaction is 0°C to 200°C, and is preferably 25°C to 200°C. The pressure during the epoxidation reaction may be a pressure sufficient to maintain a liquid state of a reaction phase. Generally, it is advantageous that the pressure is 100 kPa to 10000 kPa.

After the epoxidation reaction ends, it is possible to separate, from a catalyst composition, a liquid mixture containing a desired product. Subsequently, it is possible to purify the liquid mixture by an appropriate method. Examples of a purifying method include distillation, extraction, and cleaning. The solvent and an unreacted olefin can be recycled for reuse.

The reaction in which the titanium-containing silicon oxide molded body produced by an aspect of the present invention is used as the catalyst can be carried out in a slurry system or fixed-bed system. In the case that a large-scale industrial operation is carried out, the fixed-bed system is preferably used. The reaction can be carried out by a batch process, a semicontinuous process, or a continuous process.

Note that the titanium-containing silicon oxide molded body obtained by the above production method and the catalyst containing the titanium-containing silicon oxide molded body are also included in the scope of the present invention.

The titanium-containing silicon oxide molded body in accordance with an aspect of the present invention preferably satisfies the following requirements (1) and (2).

(1) Not less than 90% of the total pore volume has a pore size of 5 Å to 200 Å.
(2) Specific pore volume is not less than 0.2 $cm^3/g$.

Note, here, that the specific pore volume means pore volume per gram of the catalyst.

Measurement for the requirements (1) and (2) can be carried out by a typical method with use of a physical adsorption method in which a gas such as nitrogen or argon is used (gas absorption method).

Note also that, as used herein, a "conversion of the hydroperoxide" by the titanium-containing silicon oxide molded body is a value calculated by the following procedure and calculation formula.

To an autoclave, 0.5 g of the titanium-containing silicon oxide molded body obtained by an aspect of the present invention, 60 g of a solution in which CMHP is dissolved in CUM at a concentration of 25% by mass, and 33 g of propylene are fed. These are then reacted with each other under a self-generative pressure at a reaction temperature of 100°C for reaction time of 1.5 hours (including heat-up time).

$$\text{The conversion of the hydroperoxide (\%)} = M_1/(M_0-M_1) \times 100$$

$M_0$: A molar quantity of the hydroperoxide, which is a raw material

$M_i$: A molar quantity of the hydroperoxide in a solution after the reaction

Here, it is preferable that the titanium-containing silicon oxide molded body in accordance with an aspect of the present invention has a conversion of the hydroperoxide, which conversion is an index for catalytic activity, of not less than 80%.

The titanium-containing silicon oxide molded body in accordance with an aspect of the present invention has high catalytic activity and high strength of a titanium-containing silicon oxide (powdering ratio is low). Therefore, in the case that the titanium-containing silicon oxide is used as the catalyst, it is possible to prevent loss of pressure loss. In an industrial operation, the titanium-containing silicon oxide molded body can suitably prevent an increase in amount of fine powder derived from the catalyst with time due to a continuous operation, and an accompanying increase in pressure loss with the time.

[Recap]

The present invention provides the following [1] through [9].

[1] A method for producing a titanium-containing silicon oxide molded body, including:

a raw material mixing step of mixing a templating agent, a silicon source, and a solvent to obtain a solid containing the templating agent and a silicon oxide;
a molding step of molding the solid, obtained through the raw material mixing step, to obtain a molded body, the solid being molded under a condition that a temperature of the solid is 40°C to 100°C; and
a templating agent removing step of removing the templating agent from the molded body, obtained through the molding step, to obtain the molded body from which the templating agent has been removed,
in and/or after any of the raw material mixing step, the molding step, and the templating agent removing step, titanium being introduced into the solid or the molded body.

[2] The method as described in [1], wherein the templating agent is a surfactant.
[3] The method as described in [1], wherein the templating agent is piperidine in which at least one hydrogen atom may be substituted by a hydrocarbon group having 1 to 10 carbon atoms.

[4] The method as described in [2], wherein the templating agent is a quaternary ammonium compound containing a quaternary ammonium ion represented by the following Formula (I) or is an amine represented by the following Formula (II):

$$[NR^1R^2R^3R^4]^+ \qquad (I)$$

where: $R^1$ represents a hydrocarbon group having 2 to 36 carbon atoms; and $R^2$ to $R^4$ each independently represent a hydrocarbon group having 1 to 6 carbon atoms; and

$$NR^5R^6R^7 \qquad (II)$$

where: $R^5$ represents a hydrocarbon group having 2 to 36 carbon atoms; and $R^6$ and $R^7$ each independently represent a hydrogen atom or a hydrocarbon group having 1 to 6 carbon atoms.

[5] The method as described in any one of [1] through [4], wherein introduction of the titanium is carried out in the raw material mixing step.

[6] The method as described in any one of [1] through [5], wherein a molding method employed in the molding step is a compression method.

[7] The method as described in any one of [1] through [6], wherein, in the templating agent removing step, the templating agent is removed by extraction with use of a solvent.

[8] A method for producing an epoxide, including reacting an olefin with a hydroperoxide in the presence of a catalyst containing a titanium-containing silicon oxide molded body produced by the method described in any one of [1] through [7].

[9] A titanium-containing silicon oxide molded body produced by a method including:

a raw material mixing step of mixing a templating agent, a silicon source, and a solvent to obtain a solid containing the templating agent and a silicon oxide;

a molding step of molding the solid, obtained through the raw material mixing step, to obtain a molded body, the solid being molded under a condition that a temperature of the solid is 40°C to 100°C; and

a templating agent removing step of removing the templating agent from the molded body, obtained through the molding step, to obtain the molded body from which the templating agent has been removed,

in and/or after any of the raw material mixing step, the molding step, and the templating agent removing step, titanium being introduced into the solid or the molded body.

The present invention is not limited to the embodiments, but can be altered by a skilled person in the art within the scope of the claims. The present invention also encompasses, in its technical scope, any embodiment derived by combining technical means disclosed in differing embodiments.

Examples

Hereinafter, one embodiment of the present invention will be described in more detail with reference to Examples.

[Example 1]

(A) Raw material mixing step and introduction of titanium

With a mixed solvent having a mixing ratio (mass ratio) of water:methanol = 72:28, hexadecyl trimethylammonium hydroxide was diluted so that a concentration of the hexadecyl trimethylammonium hydroxide became 16% by mass. The hexadecyl trimethylammonium hydroxide (277 parts by mass in terms of an amount of the solution having a concentration of 16% by mass) was stirred. While the hexadecyl trimethylammonium hydroxide was being stirred, a mixed solution of 4 parts by mass of tetraisopropyl titanate and 10 parts by mass of 2-propanol was added to the hexadecyl trimethylammonium hydroxide by dropping at 40°C. After the dropping had been completed, a resultant mixture was stirred for 30 minutes at 40°C. Subsequently, while the mixture was being stirred, 84 parts by mass of tetramethyl orthosilicate was dropped in the mixture at 40°C. Thereafter, stirring was continued at 40°C for 3 hours, and a produced solid was filtered out. The obtained solid was dried under a reduced pressure at 70°C. Note that the hexadecyl trimethylammonium hydroxide, the tetramethyl orthosilicate, and the tetraisopropyl titanate are a templating agent, a silicon source, and a titanium source, respectively.

(B) Molding step

To 100 parts by mass of a white solid obtained in the raw material mixing step, water was added so that the white solid had a water content of 13 parts by mass. The white solid and the water were mixed well. A resultant mixture was compression-molded at a pressure of 1.3 ton/cm$^2$ with use of a tablet molding machine which was heated. A temperature of the solid thus compressed was measured with use of a contact thermometer. The temperature of the solid was 40°C. The solid thus obtained was crushed, and a crushed solid was sieved to obtain a molded body having a particle diameter of 1.0 mm to 2.0 mm.

(C) Templating agent removing step

In a flask, 3 g of the molded body obtained in the molding step was put. A mixed solution of 30 ml of methanol and 1.5 g of concentrated hydrochloric acid (a content of 36% by weight) was then added to the molded body. This flask was put in an oil bath at 70°C, and the flask was heated for 1 hour. The flask was then let to stand to cool. Subsequently, a solution was removed by decantation. A similar operation was repeated again with use of a mixed solution of 30 ml of methanol and 0.7 g of concentrated hydrochloric acid. After 30 ml of methanol was further added to the flask and then the flask was heated for 1 hour in an oil bath at 70°C, a solution was removed by decantation. The flask was put in an oil bath at 120°C under a reduced pressure of 10 mmHg, and a solvent was dried for 1.5 hours so that the solvent was removed. As a result, the molded body from which the templating agent had been removed was obtained.

(D) Silylating step

To the above molded body from which the templating agent had been removed, a mixed solution of 1 g of 1,1,1,3,3,3-hexamethyldisilazane and 15 g of toluene was added. The flask was put in an oil bath at 120°C, and the flask was heated for 1.5 hours. The flask was then let to stand to cool. Subsequently, a solvent was removed by decantation. The flask was put in an oil bath at 120°C under a reduced pressure of 10 mmHg, and the molded body was dried for 1.5 hours. As a result, a titanium-containing silicon oxide molded body was obtained. A range of pore distribution of the titanium-containing silicon oxide molded body by a nitrogen absorption method was 5 Å to 80 Å. Specific pore volume of the titanium-containing silicon oxide molded body was 0.89 ml/g.

[Example 2]

A molded body and a titanium-containing silicon oxide molded body were each obtained in a manner similar to that in Example 1, except that, in the molding step in Example 1, (i) a solid to be molded and a tablet molding machine were preheated and (ii) the solid was molded under a condition that a temperature of the solid in a compressed state was 100°C. A range of pore distribution was 5 Å to 80 Å. Specific pore volume was 0.94 ml/g.

[Comparative Example 1]

A molded body and a titanium-containing silicon oxide molded body were each obtained in a manner similar to that in Example 1, except that, in the molding step in Example 1, (i) a solid to be molded and a tablet molding machine were preheated and (ii) the solid was molded under a condition that a temperature of the solid in a compressed state was 150°C. A range of pore distribution was 5 Å to 80 Å. Specific pore volume was 0.70 ml/g.

[Example 3]

In the molding step in Example 1, a solid was molded at a linear pressure of 4 ton/cm with use of a roll press machine which was heated. A temperature of a molded body discharged from the roll press machine was measured with use of a contact thermometer. The temperature of the molded body was 40°C. The molded body and a titanium-containing silicon oxide molded body were obtained under conditions similar to those in Example 1, except that the roll press machine was used. A range of pore distribution was 5 Å to 80 Å. Specific pore volume was 1.05 ml/g.

[Example 4]

A molded body and a titanium-containing silicon oxide molded body were each obtained in a manner similar to that in Example 3, except that, in the molding step in Example 3, a solid was molded under a condition that a temperature of the molded body discharged from a roll press machine was 62°C. A range of pore distribution was 5 Å to 100 Å. Specific pore volume was 1.07 ml/g.

[Comparative Example 2]

A molded body and a titanium-containing silicon oxide molded body were each obtained in a manner similar to that in Example 3, except that, in the molding step in Example 3, a solid was molded with use of a roll press machine at a room temperature under a condition that a temperature of the molded body discharged from the roll press machine was 23°C. A range of pore distribution was 5 Å to 80 Å. Specific pore volume was 1.10 ml/g.

(E) Evaluation of strength of molded body obtained in molding step (measurement of powdering ratio)

Onto a sieve having a mesh size of 1 mm, 1.5 g of the molded body obtained in the molding step in Example 1 was collected, and a side of the sieve was strongly knocked for 30 seconds so that a fine powder was removed. In a 2 ml-syringe (having an inner diameter of 0.9 cm), 1 g of the molded body from which the fine powder had been removed was put. The 2 ml-syringe was tapped so that the molded body was tightly packed. A load of 6.0 kgf/cm$^2$ was imposed on a plunger of the 2 ml-syringe for 1 minute. Onto a sieve having a mesh size of 0.5 mm, the molded body to which the load had been imposed was collected, and a side of the sieve was strongly knocked for 30 seconds. A weight of a fine powder which had passed through a sieve mesh was measured, and a powdering ratio was calculated by the following calculation formula. A result is shown in Table 1.

$$F = W1/W2 \times 100$$

F: A powdering ratio (%)
W1: Mass of particles each of which had passed through the sieve mesh and each of which had a size of less than 0.5 mm (g)
W2: Mass of the molded body with which the 2 ml-syringe was filled (g)

Also in Examples 2 through 4 and Comparative Examples 1 and 2, powdering ratios were similarly measured. Results are shown in Table 1.

The low powdering ratio of a molded body obtained in the molding step means that the molded body obtained in the molding step has high strength. In addition, if the powdering ratio of the molded body obtained in the molding step is low, strength of the molded body from which a templating agent has been removed in the templating agent removing step and strength of a titanium-containing silicon oxide molded body obtained in the silylating step are also relatively high.

(F) Evaluation of catalytic performance (production of propylene oxide)

Catalytic performance of the titanium-containing silicon oxide molded body obtained in the silylating step in Example 1 was evaluated with use of a batch-type chemical reactor (autoclave). To the autoclave, 0.5 g of the titanium-containing silicon oxide molded body, 60 g of a solution in which CMHP was dissolved in CUM at a concentration of 25% by mass, and 33 g of propylene were fed. These were then caused to react with each other under a self-generative pressure at a reaction temperature of 100°C for reaction time of 1.5 hours (including heat-up time). A result of a reaction is shown in Table 2. Note that a "conversion" in Table 2 refers to a "conversion of CMHP (%)" (later described).
The conversion of the CMHP was calculated as follows.

$$\text{The conversion of the CMHP} = M_1/(M_0 - M_1) \times 100$$

Mo: A molar quantity of the CMHP, which is a raw material
Mi: A molar quantity of the CMHP in a solution after the reaction

Also in Examples 2 through 4 and Comparative Examples 1 and 2, conversions were similarly measured. Results are shown in Table 2.

[Table 1]

| | Example 1 | Example 2 | Example 3 | Example 4 | Comparative Example 1 | Comparative Example 2 |
|---|---|---|---|---|---|---|
| Molding temperature | 40°C | 100°C | 40°C | 62°C | 150 °C | 23 °C |
| Powdering ratio | 1% | 1% | 2% | 1% | 1% | 4% |

[Table 2]

| | Example 1 | Example 2 | Example 3 | Example 4 | Comparative Example 1 | Comparative Example 2 |
|---|---|---|---|---|---|---|
| Molding temperature | 40°C | 100°C | 40°C | 62°C | 150°C | 23 °C |
| Conversion | 94% | 89% | 94% | 95% | 76% | 94% |

Industrial Applicability

A method for producing a titanium-containing silicon oxide molded body in accordance with an aspect of the present invention can be applied to production of a catalyst which has high strength and which is used in a reaction in which an epoxide is produced from an olefin and a hydroperoxide. A titanium-containing silicon oxide molded body obtained by the method can be used for, for example, production of a propylene oxide as a catalyst.

**Claims**

1.   A method for producing a titanium-containing silicon oxide molded body, comprising:

> a raw material mixing step of mixing a templating agent, a silicon source, and a solvent to obtain a solid containing the templating agent and a silicon oxide;
> a molding step of molding the solid, obtained through the raw material mixing step, to obtain a molded body; and
> a templating agent removing step of removing the templating agent from the molded body, obtained through the molding step, to obtain the molded body from which the templating agent has been removed,
> in the molding step, the solid obtained through the raw material mixing step being molded under a condition that a temperature of the solid is 40°C to 100°C,
> in and/or after any of the raw material mixing step, the molding step, and the templating agent removing step, titanium being introduced into the solid or the molded body.

2.   The method as set forth in claim 1, wherein the templating agent is a surfactant.

3.   The method as set forth in claim 1, wherein the templating agent is piperidine in which at least one hydrogen atom may be substituted by a hydrocarbon group having 1 to 10 carbon atoms.

4.   The method as set forth in claim 2, wherein the templating agent is a quaternary ammonium compound containing a quaternary ammonium ion represented by the following Formula (I) or is an amine represented by the following Formula (II):

$$[NR^1R^2R^3R^4]^+ \qquad (I)$$

where: $R^1$ represents a hydrocarbon group having 2 to 36 carbon atoms; and $R^2$ to $R^4$ each independently represent a hydrocarbon group having 1 to 6 carbon atoms; and

$$NR^5R^6R^7 \qquad (II)$$

where: $R^5$ represents a hydrocarbon group having 2 to 36 carbon atoms; and $R^6$ and $R^7$ each independently represent a hydrogen atom or a hydrocarbon group having 1 to 6 carbon atoms.

5. The method as set forth in any one of claims 1 through 4, wherein introduction of the titanium is carried out in the raw material mixing step.

6. The method as set forth in any one of claims 1 through 5, wherein a molding method employed in the molding step is a compression method.

7. The method as set forth in any one of claims 1 through 6, wherein, in the templating agent removing step, the templating agent is removed by extraction with use of a solvent.

8. A method for producing an epoxide, comprising reacting an olefin with a hydroperoxide in the presence of a catalyst containing a titanium-containing silicon oxide molded body produced by the method recited in any one of claims 1 through 7.

9. A titanium-containing silicon oxide molded body produced by a method comprising:

a raw material mixing step of mixing a templating agent, a silicon source, and a solvent to obtain a solid containing the templating agent and a silicon oxide;
a molding step of molding the solid, obtained through the raw material mixing step, to obtain a molded body; and
a templating agent removing step of removing the templating agent from the molded body, obtained through the molding step, to obtain the molded body from which the templating agent has been removed,
in the molding step, the solid obtained through the raw material mixing step being molded under a condition that a temperature of the solid is 40°C to 100°C,
in and/or after any of the raw material mixing step, the molding step, and the templating agent removing step, titanium being introduced into the solid or the molded body.

## INTERNATIONAL SEARCH REPORT

| International application No. |
|---|
| PCT/JP2019/010060 |

### A. CLASSIFICATION OF SUBJECT MATTER
Int.Cl. B01J21/08(2006.01)i, B01J37/00(2006.01)i, B01J37/03(2006.01)i, C01B33/12(2006.01)i, C07D301/14(2006.01)i, C07B61/00(2006.01)n

According to International Patent Classification (IPC) or to both national classification and IPC

### B. FIELDS SEARCHED

Minimum documentation searched (classification system followed by classification symbols)
Int.Cl. B01J21/08, B01J37/00, B01J37/03, C01B33/12, C07D301/14, C07B61/00

| Documentation searched other than minimum documentation to the extent that such documents are included in the fields searched | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2006-255586 A (SUMITOMO CHEMICAL CO., LTD.) 28 September 2006, claims 1-9, paragraphs [0051]-[0054], [0068]-[0081] (Family: none) | 1-9 |
| A | JP 2012-131696 A (SUMITOMO CHEMICAL CO., LTD.) 12 July 2012, claims 1-9, paragraphs [0002], [0007], [0010], [0065]-[0075] & WO 2012/074033 A1 | 1-9 |
| A | JP 2017-505223 A (LYONDELL CHEMICAL TECHNOLOGY, L.P.) 16 February 2017, claims 1-20 & US 2015/0375200 A1, claims 1-20 & WO 2015/112672 A1 & EP 3096879 A1 | 1-9 |
| A | CN 104340988 A (CHINA PETROLEUM & CHEMICAL CORPORATION SINOPEC CORP., RESEARCH INSTITUTE OF PETROLEUM PROCESSING) 11 February 2015, claims 1-11 (Family: none) | 1-9 |

☐ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 30 May 2019 (30.05.2019) | 11 June 2019 (11.06.2019) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006159057 A

- US 5143879 A